(19) **Europäisches Patentamt / European Patent Office / Office européen des brevets**

(11) **EP 1 523 988 B1**

(12) **EUROPEAN PATENT SPECIFICATION**

(45) Date of publication and mention of the grant of the patent:
**23.07.2008 Bulletin 2008/30**

(51) Int Cl.:
*A61K 36/344* (2006.01)  *A61K 36/481* (2006.01)
*A61P 37/02* (2006.01)  *A61P 35/00* (2006.01)
*A61P 9/10* (2006.01)  *A61P 7/02* (2006.01)
*A61P 11/00* (2006.01)

(21) Application number: **04702624.0**

(22) Date of filing: **16.01.2004**

(86) International application number:
**PCT/CN2004/000056**

(87) International publication number:
**WO 2004/096249 (11.11.2004 Gazette 2004/46)**

(54) **COMPOSITION COMPRISING RADIX CODONOPSIS PILOSULAE AND RADIX ASTRAGALI FOR THE TREATMENT OF ACUTE LUNG INJURY**

ZUSAMMENSETZUNG MIT RADIX CODONOPSIS PILOSULAE UND RADIX ASTRAGALI ZUR BEHANDLUNG VON AKUTEN LUNGENVERLETZUNGEN

COMPOSITION CONTENANT RADIX CODONOPSIS PILOSULAE ET RADIX ASTRAGALI POUR LE TRAITEMENT DE LESIONS PULMONAIRES AIGUES

(84) Designated Contracting States:
**AT BE BG CH CY CZ DE DK EE ES FI FR GB GR HU IE IT LI LU MC NL PT RO SE SI SK TR**

(30) Priority: **29.04.2003   CN 03123045**
**18.06.2003   CN 03137351**
**02.09.2003   CN 03156284**

(43) Date of publication of application:
**20.04.2005   Bulletin 2005/16**

(73) Proprietor: **Li Min Pharmaceutical Factory of Livzon Pharmaceutical Group**
**Shaoguan,**
**Guangdong 512028 (CN)**

(72) Inventors:
- **XIE, Weihong**
**Shaoguan,**
**Guangdong 512028 (CN)**
- **ZHI, Chunhan**
**Shaoguan,**
**Guangdong 512028 (CN)**
- **TAO, Desheng**
**Shaoguan,**
**Guangdong 512028 (CN)**

(74) Representative: **Grünecker, Kinkeldey, Stockmair & Schwanhäusser Anwaltssozietät**
**Leopoldstrasse 4**
**80802 München (DE)**

(56) References cited:
**CN-A- 1 089 146**  **CN-A- 1 097 330**
**CN-A- 1 217 204**  **CN-A- 1 266 707**
**CN-A- 1 362 102**  **CN-A- 1 393 257**
**CN-A- 1 415 341**  **CN-A- 1 456 242**
**CN-A- 1 456 262**

- **XU Z-B ET AL: "RADIX CODONOPSIS PILOSULAE AND RADIX ASTRAGALI PROMOTE ERYTHROCYTE GLYCOLYSIS" CHINESE MEDICAL JOURNAL (ENGLISH EDITION), vol. 100, no. 8, 1987, pages 654-657, XP009070128 ISSN: 0366-6999**
- **LIAO J Z ET AL: "Pharmacologic effects of codonopsis pilosula-astragalus injection in the treatment of CHD patients." JOURNAL OF TRADITIONAL CHINESE MEDICINE = CHUNG I TSA CHIH YING WEN PAN / SPONSORED BY ALL-CHINA ASSOCIATION OF TRADITIONAL CHINESE MEDICINE, ACADEMY OF TRADITIONAL CHINESE MEDICINE. MAR 1988, vol. 8, no. 1, March 1988 (1988-03), pages 1-8, XP009070132 ISSN: 0254-6272**

- **LI W ET AL: "EFFECTS OF CODONOPSIS-PILOSULA ASTRAGALUS INJECTION ON PLATELET AGGREGATION AND ACTIVITY OF PROSTAGLANDIN I-2-LIKE SUBSTANCE" JOURNAL OF TRADITIONAL CHINESE MEDICINE, vol. 6, no. 1, 1986, pages 9-12, XP009070134 ISSN: 0254-6272**

## Description

### Field of the Invention

The present invention relates to a composition of Radix Codonopsis (Fllase Asiabell Root Tangshen) and Radix Astragali (Milkvetch Root), of which the main component is prepared according to a certain weight ratio of Radix Codonopsis and Radix Astragali. The invention also provides a method of preparing the pharmaceutical composition and its applications in the preparation of medicaments for the treatment of acute lung injury.

### Background of the Invention

Digestive tract tumor is a popular tumor in China. As it is difficult to be identified in time, the early diagnostic rate is relatively low. It not only makes people lose precious operation chances, but also causes intolerance of chemical medicaments because of low immunological functioning that is caused by excessive pathogen but weak vital QI, and blood stagnancy due to QI deficiency.

It has become a very intractable clinical problem in recent years.

At present, there are many ways for treating tumors. It is well known that the treatments with Western medicines such as cyclophosphamide, methotrexate, 5-fluorouracil, cis *platin* or doxorubicin have apparent toxic side effects on normal body cells because they inhibit the growth of tumor cells mainly by inhibiting synthesization of nucleic acid or tumor cells protein. Therefore, they will lead to some symptoms such as nausea, vomit, marrow inhibition, etc. that many patients cannot tolerate chemotherapy and have to interrupt their treatments. More seriously, it might cause death to some patients. In addition, the occurrence and development of tumors closely relate to the human immunological function, especially with the cells immunological function. Thus, an ideal immunomodulator is expected to be found to enhance the stress capability of the whole body, maintain the immunocompetence of human body in the process of chemotherapy, reduce toxic side effects and extend the survival period of patients.

There are also researches of using traditional Chinese medicine as immunomodulator modifier. For example, Xie Yan, *et.al.* have reported an injection containing the components of Panax ginseng and Radix Astragali in ZL94101456.8, which is used to enhance the immunological functioning of human and inhibit the occurrence and development of tumors. However, experimental data, which could prove the medicaments' effects, is not provided in that Patent. Furthermore, the pharmaceutical component Panax ginseng is too expensive which could be a heavy burden to the patients who have to use the medicament for a long period of time and make patients give up the treatments for financial reasons. To solve this problem, the inventors systematically studied the action system of the medical composition in immunoregulation and accidentally found that the composition of Radix Codonopsis and Radix Astragali described in the present invention had other useful applications. For example, the applicant had found that the composition of Radix Codonopsis and Radix Astragali has definite treating effects in treatment of acute pulmonary injury. And those treating effects have been proved by experimental data.

### Summary of the Invention

The present invention relates to *the use* of a composition with pharmaceutical values, which is extracted from the traditional Chinese medicine of Radix Codonopsis and Radix Astragali *for the preparation of a medicament for preventing and treating the acute pulmonary injury.*

The present invention also relates to a method of preparing the pharmaceutical composition.

A composition of the present invention is mainly made of Radix Codonopsis and Radix Astragali as raw materials.

The preferred ratio of Radix Codonopsis and Radix Astragali is in the range of 0.5:1 to 1:0.5; and the more preferred is 1:1.

Traditional Chinese medicines with effects of reinforcing QI and enriching blood could also be included in raw materials of the composition of the present invention, such as Radix Angelicae Sinensis, Radix rehmanniae Praeparata, Radix Polygoni Multiflori, Rhizoma Atractylodis Macrocephalae, Rhizoma Dioscoreae, etc.

The method of preparing the composition of the present invention has the following steps:

a) Impurities were removed from Radix Codonopsis and Radix Astragali and the purified materials were made into herbal pieces prepared for decoction;
b) Radix Codonopsis and Radix Astragali were taken according to a certain weight ratio and washed with deionized water;
c) A certain amount of deionized water was added *stepwise* according to the mass of Radix Codonopsis and Radix Astragali; materials were then heated and extracted 1 - 3 times to obtain the pharmaceutical extract;
d) The pharmaceutical extract was condensed and the condensed liquid was obtained;

e) Appropriate amount of ethanol was added to precipitate normally. The liquid was filtrated. The ethanol from the filtered liquid was recovered, condensed and dried to get the extracted composition of Radix Codonopsis and Radix Astragali.

In Step c), when the medicaments were extracted by adding water, the amount of added water was preferred 8 times volume at the first time and water was decocted for one hour; then 6 times volume of water was added at the second time and decocted for 0.5 hour.

In Step e), when the medicament was precipitated by adding ethanol, it was preferred that the amount of ethanol was 65%-80% of the total amount at the first time; and the amount of ethanol was not less than 80% of the total amount at the second time.

The traditional Chinese medicine of Radix Codonopsis of the present invention is the dried root of Codonopsis Pilosula (Franch.) Nannf; the traditional Chinese medicine of Radix Astragali is the dried root of Astragalus membranaceus (Fisch.) Bge. or Astragalus membranaceus (Fisch.) Bge. Var. mongholicus (Bge.) Hsiao.

For every gram of the composition provided in the invention, there contains solid no less than 0.325 g. Its main components are saccharides (including polysaccharide and monosaccharide), organic acid, saponin, coumarin extract (little), flavone glycoside, alkaloid, steroid, paraffin hydrocarbon, and etc. The main active constituents are polysaccharide, saponin, coumarin extract and flavone glycoside.

The present invention relates further to the application of the composition of Radix Codonopsis and Radix Astragali in preparation of a medicament for treating acute pulmonary injury.

The composition can be in the form of injection, tablet, pill, capsule, granule, solution, suspension, and emulsion. The mass range of effective dose of the composition is 58 - 70mg/kg (body weight)/day.

In the application, one or more pharmaceutically acceptable carriers can be added in the medicament of the present invention to prepare needed forms, such as diluent, excipient, filling, binder, wetting agent, disintegrator, sorbefacient, surface-active, adsorption carrier, lubricant etc.

In accordance with actual needs, the medicament of the present invention can be further prepared in many forms such as oral liquid, tablet, capsule, granule and injection. All of the forms of prepared drugs can be prepared in accordance with the conventional methods in the pharmacy area.

**Brief Description of the Drawing**

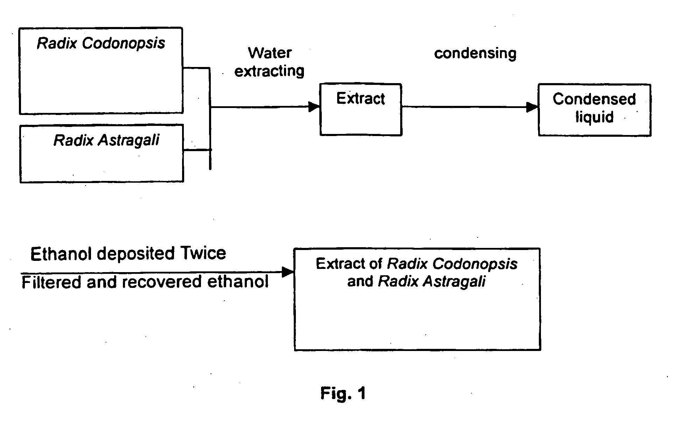

Fig.1 shows the extracting technology of the Traditional Chinese medicine of Radix Codonopsis and Radix Astragali.

**Preferred Embodiments of the Invention**

The composition of the invention, the method for preparation thereof and its application will be further described with the following embodiments and Experimental Examples.

**Example 1:**

**Radix Codonopsis and Radix Astragali were extracted and separated to obtain the composition of Radix Codonopsis and Radix Astragali.**

Impurities were removed from Radix Codonopsis and Radix Astragali respectively and the two medicaments were made into herbal pieces prepared for decoction. 400 grams of Radix Codonopsis and Radix Astragali were exactly weighed respectively and washed with deionized water. 3200 ml deionized water was added and the solution was heated for 1 hour. Then the extraction liquid was let out. Next, an extraction liquid was obtained by adding 2400 ml deionized water and heating for 1 hour. Next, the extraction liquid was obtained by adding 2400 ml deionized water and heating for 0.5 hour. The three extractions liquid were filtered, mixed, and condensed into 600 ml, and added 95% ethanol to get the ethanol concentration of 60%. Filtrate was recovered to 400 ml after depositing 24 hours, and 95% ethanol was then added to increase the ethanol concentration to 80%. The extracted composition of Radix Codonopsis and Radix Astragali was obtained 400 grams by depositing, filtering and condensing the recovered ethanol.

Appropriate amount of ethanol could be added to the medicament of the present invention for cold storage. The medicament could also be stored at room temperature after being dried. The solid was no less than 0.325g per gram of the medicament. Its main components were saccharide (including polysaccharide and monosaccharide), organic acid, saponin, coumarin extract (little), flavone glycoside, alkaloid, steroid, paraffin hydrocarbon, etc., among which the main active constituents were polysaccharide, saponin, coumarin extract and flavone glycoside.

The useful effect of the said medicament of the invention will be further described in the following experimental examples. These experimental examples include the animal and clinical observation experiments of the composition of the invention.

**Reference Example 1:**

**The antitumor effect of the composition of Radix Codonopsis and Radix Astragali and Influence to the lethal effect of MTX (Methotrexate)**

**1. Experimental Medicament**

The composition of Radix Codonopsis and Radix Astragali was the extracted composition of Radix Codonopsis and Radix Astragali prepared in Example 1 mentioned above in which there was 3.25g extract of Radix Codonopsis and Radix Astragali per 20 ml. It was then condensed to 2ml - 1 ml by water bath and sterilized by steam in the experiment.
MTX was produced by Shanghai Second Pharmaceutical Factory with the Product Number 870615. It was prepared to 1mg/ml aqueous solution in the experiment.

**2. Experimental animal:**

Female Kunming Hybrid mice were provided by the Animal House of China Research Institution with the body weight of 18 - 22g.

**3. Experimental Method:**

1) The mice were randomly divided into high dose, low dose and control groups. Sarcoma 180 lines of mice were used as the tumor lines. The fresh tumor tissues were made into cell plasma solution under sterile conditions and diluted with a ratio of 1:4. Each mouse was subcutaneously inoculated under the right armpit with 0.15ml solution and was given medicaments after 24 hours. In the high dose group, 0.4 ml of the condensed liquid of the composition of Radix Codonopsis and Radix Astragali was given per mouse; in *the* low dose group, 0.2ml of the condensed liquid of the composition was given per mouse; and for the control group, 0.4 ml of physiological saline was given per mouse. All mice were injected intraperitoneally daily for 14 days. On the 15th day, all the animals were killed. The tumors were weighed to find the inhibition rate of tumor weight. The data were statistically analyzed with the t Test Method.
2) The mice were divided into two groups and inoculated the tumor cells according to the method above. At the 3rd days before inoculation, the administration group and the control group were respectively injected intraperitoneally with the extracted composition of Radix Codonopsis and Radix Astragali and the physiological saline 0.2ml per rat daily for continuously eight days. On the 4th day of the administration, the mice were inoculated tumor cell and intraperitoneally injected 22mg/kg aqueous solution of MTX after 6 hours of inoculation. Within 10 days after MTX injection, the death rate of animals was observed and the survival rates of the two groups of animals were compared.

**4. Experimental Results:**

The results of the influence of the extracted composition of Radix Codonopsis and Radix Astragali on Sarcoma 180 lines of mice could be found in Table 1 and Table 2.

Table 1: The influence of the extracted composition of Radix Codonopsis and Radix Astragali (High dose group) on the Sarcoma 180 lines of mice.

| No. of Tests | Groups | Dose (g/kg) | Number. of Animals | Tumor weight (X±SD) | Tumor Inhibition Rate | P Value |
|---|---|---|---|---|---|---|
| 1 | Control Group | 6.50 | 21 | 3.95±2.38 | 36.7 | 0.01<P<0.05 |
| | High dose group | | 20 | 2.50±1.66 | | |

(continued)

| No. of Tests | Groups | Dose (g/kg) | Number. of Animals | Tumor weight (X±SD) | Tumor Inhibition Rate | P Value |
|---|---|---|---|---|---|---|
| 2 | Control Group | 6.50 | 14 | 3.24±1.43 | 55.28 | P<0.01 |
| | High dose group | | 8 | 1.53±0.74 | | |
| 3 | Control Group | 6.50 | 21 | 5.97±2.95 | 33.84 | 0.01<P<0.05 |
| | High dose group | | 21 | 3.95±2.63 | | |

Table 2 : The influence of the extracted composition of Radix Codonopsis and Radix Astragali (low dose group) on Sarcoma 180 lines of mice

| No. of Tests | Groups | Dose (g/kg) | Number. of Animals | Tumor weight (X±SD) | Tumor Inhibition Rate | P Value |
|---|---|---|---|---|---|---|
| 1 | Control Group | 3.25 | 20 | 2.97±1.72 | 20.86 | P>0.05 |
| | Low dose group | | 9 | 2.21±1.09 | | |
| 2 | Control Group | 3.25 | 14 | 3.42±1.43 | 23.40 | P>0.05 |
| | Low dose group | | 7 | 2.62±1.67 | | |
| 3 | Control Group | 3.25 | 15 | 3.89±1.22 | 19.69 | P>0.05 |
| | Low dose group | | 12 | 3.12±0.66 | | |

From Table 1 and Table 2, we could see that the average tumor weights of the two administration groups were both less than that of the control group after 14 days of administration. In the three experiments of the 6.50 g extraction/kg group, the tumor inhibiting rates are 36.7%, 55.28% and 33.84% respectively. The data demonstrate significant difference between the two administration groups and the control group after being statistically analysed. In the three experiments of the 3.25g extraction/kg groups, the tumor inhibiting rates were 20.86%, 23.40% and 19.69% respectively. Those data indicated that the extracted composition of Radix Codonopsis and Radix Astragali had an inhibiting effect on the Sarcoma 180 lines of mice.

2) The results of the influence of the extracted composition of Radix Codonopsis and Radix Astragali upon the lethal effect of MTX could be found in Table 3.

Table 3: The influence of the extracted composition of Radix Codonopsis and Radix Astragali upon the lethal effect of MTX

| Groups | Number of animals | Number of Dead Animal after administered MTX | | | | | Survival Number after 10 days | Survival Rate % after 10 days |
|---|---|---|---|---|---|---|---|---|
| | | 4th day | 5th day | 6th day | 7th day | 8th day | | |
| MTX + the Composition Group | 13 | 2 | 1 | 2 | | 1 | 7 | 53.85※ |

(continued)

| Groups | Number of animals | Number of Dead Animal after administered MTX | | | | | Survival Number after 10 days | SurvivalRate % after 10 days |
|---|---|---|---|---|---|---|---|---|
| | | 4th day | 5th day | 6th day | 7th day | 8th day | | |
| MTX Group | 21 | 1 | 7 | 3 | 6 | | 4 | 19.04 |
| ※ Testing directly by the method of calculation of Probability: 0.01 <P<0.05. | | | | | | | | |

From Table 3, we could see that the survival rate of the composition of Radix Codonopsis and Radix Astragali group was apparently higher than that of the control group. There was significant difference in statistics analysis after tested by the Card Test of P<0.05. The experiments suggested that the extracted composition of Radix Codonopsis and Radix Astragali had a protective toxicity reducing effect upon the lethal effect of MTX, that is, it could prolong the survival period of the experiment animals.

The two experiments mentioned above proved that the extracted composition of Radix Codonopsis and Radix Astragali had an inhibiting effect on Sarcoma 180 lines of mice, could reduce the death rate of mice caused by the lethal effect of MTX, and prolonged the survival period of animals.

**Reference Example 2:**

The influence of the composition of Radix Codonopsis and Radix Astragali upon the haemopoietic system

**1. Experimental Medicament**

One injection of the composition of Radix Codonopsis and Radix Astragali was 20ml and contained equivalent 3.25g extracts of Radix Codonopsis and Radix Astragali. In the experiment, each mouse was intraperitoneally injected 0.2ml (10ml/kg/weight).

**2. Experimental animal:**

Kunming Hybrid mice with weight of 18-22g were inoculated with Sarcoma S180 lines. After 24 hours of inoculation, mice of the experimentation group were intraperitoneally injected 0.2ml injection liquid of the composition of Radix Codonopsis and Radix Astragali (containing about 0.2g crude drug), and mice of the control group were injected with an equivalent amount of water until the experiments ended. In the experiment, the cyclophosphamide was intraperitoneally injected twice consecutively, 1.5mg per mouse.

**3. Experimental Results:**

1) The influence of the injection of the composition of Radix Codonopsis and Radix Astragali upon the total number of mice medullary cells

**Table 4: The influence of the injection of the composition of Radix Codonopsis and Radix Astragali upon the total number of mice medullary cells**

| | Number of animals | $*10^6$/(M$\pm$SD) thighbone | P Value |
|---|---|---|---|
| Control group of cyclophosphamide | 10 | 9.95$\pm$3.38 | |
| Group of cyclophosphamide and the Composition | 14 | 19.98$\pm$5.21 | <0.001 |

The result in Table 4 showed that cyclophosphamide could cause the reduction of the total number of mice medullary cells. Having been combining with the injection of the composition of Radix Codonopsis and Radix Astragali, the total number of medullary cells was higher than that of the Control group.

2) The influence of the injection of the composition of Radix Codonopsis and Radix Astragali upon the medullary karyocytes of mice

**Table 5: The influence of the injection of the composition of Radix Codonopsis and Radix Astragali upon the medullary karyocytes of mice**

|  | Number of animals | $*10^6$/(M$\pm$SD) thighbone | P Value |
|---|---|---|---|
| Control group of cyclophosphamide | 10 | 5.06$\pm$1.38 | <0.01 |
| Group of cyclophosphamide and the Composition | 14 | 8.45$\pm$5.06 |  |

The medullary karyocytes represented the cells having haemopoietic functions. The result in Table 5 showed that after combining with the injection of the composition of Radix Codonopsis and Radix Astragali, the number of medullary karyocytes was apparently higher than that of the control group of only cyclophosphamide.

3) The influence of the injection of the composition of Radix Codonopsis and Radix Astragali upon the volume of medullary cells of mice

**Table 6: The influence of the injection of the composition of Radix Codonopsis and Radix Astragali upon the volume of mice medullary cells**

|  | Number of animals | M$\pm$SD volume% | P Value |
|---|---|---|---|
| Control group of cyclophosphamide | 10 | 125.90$\pm$19.26 | <0.01 |
| Group of cyclophosphamide and the Composition | 14 | 149.93$\pm$23.96 |  |

The volume of medullary cells showed the maturity of cells. The more juvenile the cell was, the bigger the volume was. The juvenile cells mostly represents the hemopoietic progenitor cell. The result of the Table 6 showed that the average volume of the group combined with the injection of the composition of Radix Codonopsis and Radix Astragali was larger than that of the control group of cyclophosphamide. (P<0.01)

4) The influence of the injection of the composition of Radix Codonopsis and Radix Astragali upon the hematocrit of medullary cells of mice

**Table 7: The influence of the injection of the composition of Radix Codonopsis and Radix Astragali upon the hematocrit of mice medullary cells**

|  | Number of animals | M$\pm$SD volume% | P Value |
|---|---|---|---|
| Control group of cyclophosphamide | 10 | 23.80$\pm$8.40 | <0.01 |
| Group of cyclophosphamide and the Composition | 14 | 34.40$\pm$11 |  |

The value of the hematocrit of medullary cells represented the size and number of the cells. The result of the Table 7 showed that the hematocrit of the group of combined with the composition of Radix Codonopsis and Radix Astragali was larger than that of control group of cyclophosphamide. (P<0.01)

**Reference Example 3:**

**The injection of the composition of Radix Codonopsis and Radix Astragali had a cooperative inhibiting effect upon tumors**

The experimental medicament and animals of this experiment were the same as that of Experimental Example 2.

Experimental Results:

The influence of the injection of the composition of Radix Codonopsis and Radix Astragali combined with cyclophosphamide upon tumor body.
The injection of the composition of Radix Codonopsis and Radix Astragali could mitigate the inhibiting effect of the mice medulla caused by chemotherapy. It was tested in this experiment whether the composition would destroy anticancer effects.

**Table 8: The influence upon tumor body by the injection of the composition of Radix Codonopsis and Radix Astragali combined with chemotherapy medication**

| | Number of animals | M±SD volume% | Rate of Inhibiting tumors | P Value |
|---|---|---|---|---|
| Blank Contrast | 9 | 2.60±1.08 | | |
| Control group of cyclophosphamide | 10 | 0.89±0.55 | 65%Δ | <0.001Δ |
| Group 1 of cyclophosphamide and the composition | 9 | 0.33±0.37 | 87%※ | <0.05※ |
| Group 2 of cyclophosphamide and the composition | 14 | 0.41±0.35 | 57%※ | <0.05※ |
| Δ Comparison between the Control group of cyclophosphamide and the Blank Control group | | | | |
| ※Comparison between the Control group of cyclophosphamide and the Control group of cyclophosphamide and the Composition of Radix Codonopsis and Radix Astragali | | | | |

The result of the Table 8 showed that the immunomodulator of Radix Codonopsis and Radix Astragali did not destroy the anticancer effect of chemotherapy. On the contrary, the rate of tumor inhibition when using the composition with the chemotherapy was much higher than when using the chemotherapy alone. In Group 1 of the composition of Radix Codonopsis and Radix Astragali, the dose was 4 times as high as the dose for human. In Group 2 of the composition of Radix Codonopsis and Radix Astragali, the dose was 2 times as high as the dose for human.

**Reference Example 4:**

**The influence of the composition of Radix Codonopsis and Radix Astragali upon the myocardial ischemia, the myocardial infarction, the related blood flow of coronary artery, the myocardial oxygen consumption and the biochemical indexes of an anesthetized dog**
30 healthy adult dogs, female and male, with body weight of 14.02±1.90 kg, were provided by Beijing TONGLI Experimental Animal Breeding Factory. (License No. 010, 2000)

**Experimental Medicament:**

The condensed liquid of the composition of Radix Codonopsis and Radix Astragali was 5ml/piece, 2g crude drug/ml. 10mg/piece Herbesser Injection (Dilitazem Hydrochloride) was produced by Tianjin TIANBIAN Pharmacy Co., Ltd. (B.N. 0003003). 0.9% sodium chloride injection was produced by Beijing. Double-crane Pharmaceutical Co., Ltd (B.N. 000320332). The injection of Radix Salviae Miltiorrhizae was 10ml/piece, 1.5g/ml and was produced by Zhengda Qingchunbao Pharmacy Co., Ltd. (Hangzhou, Zhejiang Province) (B.N. 0003132). Nitroblue tetrazole (N-BT) was produced by the Medicinal Materials Supply Station of the Military Medical Science College (B.N. 971120). Endothelin (ET) Radioimmuno -medicament Kit was produced by Beijing FURUI Bioengineering Co. (B.N. 0102). Thromboxane $B_2$ (TXB$_2$) and 6-keto-prostaglandin F1$_\alpha$ (6-Keto- PGF1$_\alpha$) Radioimmuno-medicament Kit were produced by Beijing FURUI Bioengineering Co. (B.N. 0102).

**Experimental Groups:**

(1) A Blank Control group, 3ml/kg, n=5;
(2) A Group of Herbesser Injection, 0.5ml/kg, n=5;
(3) A Group of Injection of Radix Salviae Miltiorrhizae, 0.6g/kg, n=5;
(4) A Group of the composition of Radix Codonopsis and Radix Astragali, 0.6g/kg, n=5;
(5) A Group of the composition of Radix Codonopsis and Radix Astragali, 1.2g/kg, n=5;
(6) A Group of the composition of Radix Codonopsis and Radix Astragali, 2.4g/kg, n=5.

The experimental medicaments were prepared with physiological saline to the same volume (50ml), and injected through femoral vein at a speed of 5ml/min with the computer minim syringe pump (Type AJ-5803, Shanghai).

**Experimental Methods:**

The animals were anaesthetized with pentobarbital sodium (30mg/kg, *i.v.*), and connected with an electric respirator after tracheal intubation. The chest was opened in the position of the 4th left rib to expose the heart. The pericardium was cut out to make a pericardial bed. The laevorotatory branch of coronary artery was separated and a probe of an electromagnetic flowmeter (Type MF-1100) was placed in to measure the blood flow of coronary artery. The middle section of anterior descending branch of coronary artery was separated and a thread was put through for ligature to make a model of experimental acute myocardium myocardial ischemia. A multipoint fixed epicardium electrode was sewed up and connected with a multirunning physiological recorder (Type RM-6100, Japan Photoelectricity) to display and record the electrocardiogram of epicardium[1]. The coronary artery was ligated for 15 minutes and recorded as a control value before medicaments administration. The experimental medicaments or physiological saline was injected through the femoral vein. At 5, 15, 30, 45, 60, 90, 120, and 180 minutes after medicament administration, 30 measuring points of the epicardium electrocardiogram were recorded. The rising S-T section that is more than 2mv was taken as a judgment standard to calculate the degree of myocardial ischemia (total mv number of the rising S-T section Σ - ST) and the range of myocardial ischemia (total point number of the rising S-T section N-ST). After intubation through the carotid into the coronary vein sinus, the blood oxygen content of coronary vein was measured by a blood oxygen instrument (Type AVL912, Swiss) while the blood samples were taken before ischemia, ischemia 15 min (before administration), at 15, 30, 60, 120, and 180 minutes after medicament administration. Then the animal was intubated into the carotid to measure the blood oxygen content of artery. It was calculated together with the blood flow of coronary artery to measure the myocardial oxygen consumption:
Myocardial oxygen consumption = (blood oxygen content of artery - blood oxygen content of coronary vein) x blood flow of coronary artery/100.
The blood samples were taken at the above-mentioned time points, and then serum creatine phosphate kinase (CK) and lactic dehydrogenase (LDH) were measured with a full-automatic biochemical analyzer (Type RA-1000, USA). And ET, TXB2 and 6-Keto-PGF$_1$ were measured with the Radioimmuno-method and with a full-automatic $\gamma$ counter (Type FT-630G, Beijing).

When recording was finished at 180 minutes after medicament administration, the heart was taken out immediately, washed with physiological saline and weighed as a whole. Under the ligature line of the heart, the ventricle was cut evenly into 5 pieces parallel to the coronary sulcus. Then the pieces were placed in the N-BT solution and stained at the room temperature for 15 minutes. The infarcted area (N-BT non-stained area) and the non-infarcted area (N-BT stained area) on the lateral sides of each piece of myocardium were measured with a Multimedia Color Pathological Image Analyzing System (Type MPIAS-500, Beijing) The area of each piece of myocardium, the total area of ventricle and the area of the infarcted section were calculated. The percentage of the infarcted area over the ventricle and that over total heart were calculated.

The experimental result was processed statistically and its significance was tested with t-Test.

**Experimental Results:**

**The influence upon the degree of myocardial ischemia ($\Sigma$ - ST) (recorded by the electrocardiogram of epicardium) of dogs**

The composition had influence upon the degree of myocardial ischemia ($\Sigma$ - ST) of dogs. After being administrated medicament through duodenum, both the 1.2g and 2.4g Crude Drug/kg Groups of the composition of Radix Codonopsis and Radix Astragali showed decreasing in the degree of myocardial ischemia ($\Sigma$ - ST). And the effect of the 6g Crude Drug/kg Group was better than the effect of the 3g Crude Drug/kg Group. The $\Sigma$ - ST of the 2.4g Crude Drug/kg Group of the composition of Radix Codonopsis and Radix Astragali was 293.80$\pm$97.91mv before medicament administration. The effect appeared at the point of 5 minutes after medicament administration. From 15 min to 180 min, the degree of myocardial ischemia decreased gradually. At 180 minutes, the $\Sigma$ - ST was 151.40$\pm$59.54mv with a fall of 45.70$\pm$26.23%. In comparison with the one before medicament administration and the Control group, it showed a significant difference (P<0.05 and P<0.01). The $\Sigma$ - ST of the 2.4g Crude Drug/kg Groups of the composition began to decrease at 30 minutes after medicament administration, and the medicament effect increased with the prolonged time of medicament administration. At 180 minutes, the $\Sigma$ - ST of the animals dropped from 366.80$\pm$144.99mv to 212.40$\pm$92.77mv with a fall of 40.00$\pm$18.60%. In comparison with the one before medicament administration and the Control group, it showed a significant difference (P<0.05 and P<0.01).

**The influence upon the range of myocardial ischemia (N - ST) of dogs**

When injected with physiological saline, the N-ST of the Control group didn't change obviously. In the 2.4g Crude Drug/kg Groups of the composition of Radix Codonopsis and Radix Astragali, there was an obvious effect of reducing the range of myocardial ischemia (N-ST). At 120 minutes and 180 minutes after medicament administration, the N-ST dropped from 29.40$\pm$0.89 measuring points to 26.60$\pm$2.70 and 26.20$\pm$2.28 measuring points, with a fall of 9.62$\pm$7.52% and 10.33$\pm$6.51% respectively. There was a significant difference (P<0.05) in comparison with the one before medicament administration and the Control group.

The above-mentioned results showed that the composition of Radix Codonopsis and Radix Astragali had a significant improving effect on the experimental acute myocardial ischemia of dogs. It could mitigate the degree of myocardial ischemia ($\Sigma$ - ST) and reduce the range of myocardial ischemia (N-ST).

**The influence upon the range of acute myocardial infarction of dogs (N-BT Staining Measurement) See Table 9**

**Table 9: The influence upon the range of acute myocardial infarction of dogs of all the Medicament Groups (n=5, X$\pm$SD)**

| Groups | Dose/ kg | Heart Area mm$^2$ | Ventricular Area mm$^2$ | Infarcted Area mm$^2$ | Infarcted Area/heart | Infarcted Area/ventricle |
|---|---|---|---|---|---|---|
| Physiological salt solution Herbesser | 3ml | 13185.9$\pm$ 1947.7 | 4455.2$\pm$ 985.1 | 919.80$\pm$ 224.7 | 6.94$\pm$1.2 0 | 21.53$\pm$2. 98 |
|  | 0.5mg | 11838.0 $\pm$ 2981.1 | 3883.1$\pm$8 59.8 | 281.30$\pm$ 159.9*** | 2.27$\pm$0.7 3*** | 7.44$\pm$2.6 1*** |
| Injection of Radix Salviae Miltiorrhizae | 0.6g | 11309.1 $\pm$ 1882.3 | 4622.8$\pm$ 582.2 | 487.50$\pm$ 158.0** | 4.54$\pm$2.1 4 | 10.99$\pm$3. 58** |
| Composition A | 1.5g | 11777.1 $\pm$ 2011.7 | 4188.2$\pm$ 234.0 | 556.80$\pm$ 180.1 * | 4.73$\pm$1.4 3* | 11.13$\pm$4. 24** |

(continued)

| Groups | Dose/ kg | Heart Area mm$^2$ | Ventricular Area mm$^2$ | Infarcted Area mm$^2$ | Infarcted Area/heart | Infarcted Area/ventricle |
|---|---|---|---|---|---|---|
| Composition | 3.0g | 12042. 9± 1735.3 | 4042.1± 528.1 | 478.21± 106.1** | 3.94±0.4 5** | 12.32±2. 60** |
| Composition | 6.0g | 13561. 6± 3297.9 | 4994.0* 691.8 | 484.30± 113.2** | 3.65±0.8 5*** | 10.43± 2.14*** |

**Noting:** in comparison with the Control Group: * P<0.05. ** P<0.01. *** P<0.001. The influence upon the range of acute myocardial infarction of dogs (N-BT Staining Measurement) could be seen in Table 9. The range of myocardial infarction is shown by the N-BT Staining Method of quantitative histology. The myocardial infarction area of the Control group of physiological saline occupied 6.94±1.20% and 21.53±2.98% of heart and ventricle respectively. The three groups of the Composition of Radix Codonopsis and Radix Astragali have the reducing myocardial infarction area, in which the myocardial infarction area of the 2.4g Crude Drug/kg Group occupied 3.65±0.85% and 10.43±2.14% of heart and ventricle respectively, with the decrease of 47.40% and 51.55% respectively comparing to the Control Group of physiological saline. There was a very obvious difference in comparison with the Control Group of physiological saline (P<0.001 in average). And the percentage of the myocardial infarction of heart and ventricle of Herbesser Injection Group were also obviously decreased.

### The influence upon the blood flow of coronary artery of experimental acute myocardial ischemia of dogs

After the coronary artery of anaesthetized dogs was ligated to trigger the myocardial ischemia, the blood flow of coronary artery was compensatively increased of 15% in a short period of time. It had an evident effect of increasing blood flow of coronary artery after administered Herbesser and the Composition of Radix Codonopsis and Radix Astragali. In the 2.4g Crude Drug/kg Group after medicament administration, the blood flows of coronary artery were increased during 15 - 180 minutes with the increase of 23.13±23.22% at 180 minute. There was significant difference (P<0.05) in comparison with the Group of physiological saline.

### The influence upon the blood oxygen content and the myocardial oxygen consumption of myocardium artery/ vein of experimental acute myocardial ischemia of dogs

In the Control group of physiological saline, there was no obvious change in the blood oxygen content and the myocardial oxygen consumption of artery/coronary vein sinus before and after medicament administration. In the Groups of Herbesser and the Composition of Radix Codonopsis and Radix Astragali, there was effect trend of increasing blood oxygen content of vein sinus.

However, there was no statistical meaning in it. There were no obvious changes of myocardial oxygen consumption in the three dose groups of the Composition of Radix Codonopsis and Radix Astragali. 15 minutes after given 6-Keto-PGF1, the myocardial oxygen consumption was obviously reduced.

### The influence upon the blood biochemical indexes of experimental acute myocardial ischemia of dogs

**1**. The influence upon the activity of serum creatine kinase (CK) and lactic dehydrogenase (LDH) of dogs

Before myocardial ischemia, the contents of serum CK and LDH are 491.70±201.29 u/L and 86.73±30.01 u/L (n=25) respectively. After the coronary artery was ligated to trigger the acute myocardial ischemia, the contents of CK and LDH in blood were obviously increased to the level of 669.37±239.09 u/L and 100.30±31.29 u/L respectively. Comparing with the one before myocardial ischemia, the CK and LDH content after myocardial ischemia were increased by 43.98% and 25.55% respectively. The activity of CK and LDH was further increased with the prolonged ligation. In the Groups of Herbesser and the 2.4g crude drug/kg of the composition of Radix Codonopsis and Radix Astragali, the increasing activity of CK and LDH caused by myocardial ischemia and myocardial infarction was significantly inhibited. There was significant difference (P<0.05) in comparison with the Group of physiological saline.

**2.** The influence upon the activity of endothelin (ET), thromboxane B2 (TXB2) and 6-keto-prostaglandin FI a (6-Keto-PGF1a) in plasma of dogs

In the process of continuously ligating the coronary artery of dogs, the contents of ET and TXB2 in plasma were obviously increased; and 6-Keto-PGFIa and the ratio of 6-Keto-PGFIa/ TXB2 were obviously reduced. The Herbesser and the Composition of Radix Codonopsis and Radix Astragali had an obvious inhibiting effect on the ET activity caused by myocardial ischemia and myocardial infarction. In comparison with the Group of physiological saline, there was significant difference. And at the same time, it could obviously increase 6-Keto-PGF$_{Ia}$ and the ratio of 6-Keto-PGF$_{Ia}$/ TXB$_2$.

In this experimentation, the range and degree of myocardial ischemia were measured and recorded with an epicardium electrocardiogram, the area of myocardial infarction was measured with the N-BT Staining Method, the blood flow of coronary artery, the changes of myocardial oxygen consumption, the CK and LDH activity of serum and the ET, TXB2 and 6-Keto-PGFIa activity of plasma were also measured at the same time. It was also studied the influence of the Composition of Radix Codonopsis and Radix Astragali administered through digestive tract upon the experimental acute myocardial ischemia, the myocardial infarction and related indexes of dogs.

The experimental results proved that the Composition of Radix Codonopsis and Radix Astragali had obvious effects on improving the acute myocardial ischemia and the myocardial infarction of dogs, mitigating the degree of myocardial ischemia ($\Sigma$ - ST) measured by an epicardium electrocardiogram and reducing the infarcted area displayed by N-BT Staining Method.

When myocardial ischemia and myocardial infarction occurred due to local stenosis or occlusion of coronary artery caused by different *pathogenesis,* other coronary artery branches could expand and open compensatively to mitigate myocardial ischemia and myocardial infarction. When a large area of myocardial ischemia and myocardial infarction occurred and the compensative capacity became worthless, myocardial necrosis and irreversible injury would occur which become life threats. It was observed in the experiment that the Composition of Radix Codonopsis and Radix Astragali could significantly increase the blood flow of coronary artery when myocardial ischemia and myocardial infarction occurred. It showed that it promoted opening and establishing of the lateral branch circulation, and at the same time increased oxygen supply of myocardium.

Creatine kinase (CK) existed widely in *cytoplasm,* especially in cardiac cells. When cardiac cells were injured, CK overflowed to increase its activity in serum. It was observed that the higher activity of CK in serum, the more serious of the myocardial injury. The lactic dehydrogenase (LDH) would be released greatly from histiocytes into body fluid when myocardial infarction occurred. Its activity in blood of coronary vein sinus also showed the degree of myocardial injury. It was observed in the experiment that the CK and LDH activity continued to increase when coronary artery of dogs was continuously ligated. It had been proven in the experiment that the Composition of Radix Codonopsis and Radix Astragali could partially prevent the CK and LDH overflow and reduce the CK and LDH activity in serum of experimental myocardial injury of dogs.

Prostacyclin (PGI$_2$), endothelin (ET) and thromboxane A$_2$ (TXA$_2$) were all the vascular active substances excreted from endothelial cells, in which PGI$_2$ is vasomotor; ET and TXA$_2$ were vasoconstrictor. In the experiment, active substances such as the final metabolite 6-Keto-PGF$_{Ia}$ of EK and PGI$_2$ and the metabolite TXB$_2$ of TXA$_2$ were measured to monitor the changes in the process of myocardial ischemia and myocardial infarction caused by continuously ligating coronary artery and the influence of medicaments. The result showed that the Composition of Radix Codonopsis and Radix Astragali had an obvious inhibiting effect on the increasing **TXB$_2$** activity due to myocardial ischemia and myocardial infarction, and it also at the same time enhanced the 6-Keto-PGF$_{1a}$ plasma level.

The above-mentioned results had shown that the Composition of Radix Codonopsis and Radix Astragali could obviously improve the pathologic symptoms of acute myocardial ischemia and myocardial infarction, mitigate the degree of myocardial ischemia and reduce the area of myocardial infarction.

### Reference Example 5:

**The influence of the composition of Radix Codonopsis and Radix Astragali upon myocardial infarction caused by ischemia reperfusion**

56 male Wistar mice with weight of 260 - 280g were provided by the Animal Department of Beijing General Medical Courses University with Qualification Certificate, No. 01-3056.

### Experimental Medicament:

The composition of Radix Codonopsis and Radix Astragali was 5ml/piece, 2g Crude Drug/ml. Herbesser Injection (Diltiazem Hydrochloride for Injection) was 10mg/piece and produced by Tianjin Tianbian Pharmaceutical Co.,

Ltd.. Radix Salviae Miltiorrhizae Injection was 10ml/piece, 1.5g/ml, and produced by QINGCHUNBAO Pharmaceutical Co., Ltd. (Hangzhou, Zhejiang Province) (B.N.: 0003132) Diltiazem Hydrochloride was provided by the Medicinal Materials Supply Station of the PLAAcademy of Military Medical Sciences (B.N. 971120).

**Experimental Methods:**

The animals were randomly divided into 6 groups: a Pseudo-operation Group (serum was taken as normal control), a 1.0 mg/kg Model Group, a Herbesser Group, a Group of 0.6g/kg Radix Salviae Miltiorrhizae Injection, and 8, 4, 2g Crude Drug/kg Groups of the Composition of Radix Codonopsis and Radix Astragali. The medicaments were diluted to desired concentration with physiological saline. The volume of administered medicament was 4ml/kg. And the medicaments were given through the left femoral vein.

The animals were anaesthetized by pentobarbital sodium (45mg/kg) through abdominal cavity and fixed in backstroke position. Animals were observed with Standard Lead II of an electrocardiogram (Type ECG-6511, CardiofaxX, Shanghai). The tracheas were cut open, the trachea cannulas were inserted, and tracheas were connected with a respirometer (Type SC-3, Shanghai) for

artificial respiration (32/min, with a respiration ratio of 1:3). The $3^{rd}$ - $5^{th}$ ribs were cut off after opening the chest, and the pericardiums were opened to expose the heart. A suture (suture No. 0) was put through the root of left anterior descending branch of coronary artery for ligature. After the suture being put through stably for 10 minutes, a plastic concave pipe was placed parallel with the blood vessel, which was then ligated (eliminating the ones without changes of ST and T-wave). The experimental medicaments were administered. After 40 minutes, the ligature suture was cut off along the groove to trigger the reperfusion of the anterior descending branch. The chest was then seamed and independent respiration was recovered.

The blood samples were taken from the ventral aorta to measure the serum SOD and MDA contents after 2 hours of the ligature. The heart was sliced into 5 pieces under heart ligature and was stained by N-BT. Using a Multimedia Pathological Color Image Analyzing System (Type MPIAS-500, Beijing), the areas of the normal myocardiums and the myocardial infraction were calculated. The degree of myocardial infarction was observed. And the experimental results were statistically analyzed (t-Test).

**Experimental Results:**

The influence upon the degree of myocardial infarction could be found in Table 10.

**Table 10: The influence of the Composition of Radix Codonopsis and Radix Astragali on the degree of myocardial infarction (X±S)**

| Groups | N | Dose/ kg | The area of Normal myocardium mm$^2$ | The area of myocardial infraction mm$^2$ | Weight of Infarcted Area g | Infarcted Area/ ventricle % | Infarcted Area/heart |
|---|---|---|---|---|---|---|---|
| Model | 8 | | 293.42±20.87 | 92.49±11.09 | 0.242±0.048 | 31.5±2.8 | 25.3±3.9 |
| Herbesser | 8 | 1.0mg | 287.98±23.54 | 61.72±9.68** | 0.154±0.027** | 21.6±4.3** | 16.6±2.6** |
| Radix | 8 | 1.6mg | 301.72±41.84 | 66.20±10.96** | 0.165±0.022** | 22.2±4.2** | 17.8±2.9** |
| Salviae Miltiorrhizae | | | | | | | |
| Composition | 8 | 8g | 298.31±30.99 | 66.59±8.97** | 0.186±0.020** | 22.3±1.5** | 18.9±1.6** |
| Composition | 8 | 4g | 303.12±17.23 | 66.93±7.66** | 0.179±0.029** | 22.1±2.5** | 17.6±2.7** |
| Composition | 8 | 2g | 308.18±32.31 | 74.44±8.73** | 0.206±0.027 | 25.1±2.1** | 20.86±2.3* |

*, **: in comparison with the Model group: P<0.05, P<0.01.

The experimental results proved that the percentage of the infarcted area/ventricle and heart were 31.5 and 25.3% respectively in the Model Group. The infarcted area, the weight of infarcted area and the percentage of the infarcted area/ventricle and heart were significantly reduced in the control medicaments groups of Herbesser Injection and Radix Salviae Miltiorrhizae Injection obviously. There were great difference (P<0.01) of those indexes between the control group and the medicament groups. For the 8 and 4g Crude Drug/kg Groups of the Composition of Radix Codonopsis and Radix Astragali, the infarcted area were reduced, the weight of infarcted area were mitigated, and the percentages of the infarcted area/ventricle and heart were decreased. In comparison with the Control group, there was significant difference (P<0.01). For the 2g Crude Drug/kg Group of the Composition of Radix Codonopsis and Radix Astragali, the infarcted area was reduced, the weight of infarcted area was mitigated, and the percentages of the infarcted area/ventricle and heart were decreased. There was also significant difference (P<0.05, P<0.01) comparing to the Control group.

The influence upon the contents of SOD and MDA in serum could be found in Table 11.

**Table 11: The influence of the Composition of Radix Codonopsis and Radix Astragali upon the SOD and MDA contents of blood serum (X±D)**

| Groups | Dose/kg | SOD (ng/ml) | MDA ($\mu$mol/L) |
|---|---|---|---|
| Pseudo-operation | | 643.2±167.7 | 1.93±0.41 |
| Model Group | | 492.8±53.1# | 2.95±0.46## |
| Herbesser | 1.0mg | 660.9±159.7* | 2.35±0.07** |
| Radix Salviae Miltiorrhizae | 1.6ml/kg | 586.3±92.2* | 3.55±1.91 |
| Composition | 8g/kg | 508.0±89.4 | 2.31±0.37* |
| Composition | 4g/kg | 553.5±87.5 | 2.33±0.35** |
| Composition | 2g/kg | 520.3±82.7 | 2.47±0.91 |

Note: ## Comparing to the normal Control group: P<0.01; *, ** Comparing to the Model Group: P<0.05, P<0.01.

The experimental results proved that the SOD Value was obviously reduced while the MDA Value was obviously increased. There was a significant difference (P<0.05, P<0.01) between the Model group and the Pseudo-operation Group. For the positive control medicament Herbesser, the SOD value was increased and MDA value was reduced. In the Radix Salviae Miltiorrhizae Injection Group, the SOD value was obviously increased. They all had significant difference (P<0.05 - P<0.01) in comparison with the Model Group. In the 8.0 and 4.0g/kg Groups of the Composition of Radix Codonopsis and Radix Astragali, the MDA value was obviously reduced. There was also a remarkable difference (P<0.05 - P<0.01) in comparison with the Model Group.

It was proven that after myocardium being injured because of certain period of myocardial ischemia, reperfusion could aggravate the ischemia injury, which could lead to myocardial infarction.

The effect of medicaments was observed in this experiment with the model of the myocardial ischemia reperfusion injury of rats. It was observed that the ischemia reperfusion injury led to injury of cardiac cell membrane, the SOD activity of serum of the Model Group was obviously reduced, and the MDA content was obviously increased. The symptoms mentioned above indirectly showed that the production of oxygen free radicals could further aggravate the myocardium injury.

The Composition of Radix Codonopsis and Radix Astragali could obviously reduce the area of myocardial infarction, reduce the weight of infarcted area, and have the same effect as the control medicament of Herbesser and Radix Salviae Miltiorrhizae Injection. It could markedly reduce the MDA content and protect the myocardial ischemia reperfusion injury.

### Reference Example 6:

### The influence of the Composition of Radix Codonopsis and Radix Astragali upon cardiac hemodynamics and cardiac oxygen consumption of dogs

30 healthy adult dogs, male and female, with weight of 14.10±0.22kg, were provided by Beijing TONGLI Experimental Animal Farm (with Animal License No. 010, 2000).

### Experimental Medicament:

The composition of Radix Codonopsis and Radix Astragali was 5ml/piece and 2g Crude Drug/ml. Herbesser Injection (Diltiazem Hydrochloride for Injection) was 10mg/piece and produced by Tianjin Tianbian Pharmaceutical Co., Ltd (B.N.: 0003003); 0.9% Sodium Chloride Injection was produced by Beijing Double-crane Pharmaceutical Co., Ltd (B.N.: 000320332); Injection of Radix Salviae Miltiorrhizae was 10ml/piece, 1.5g/ml and produced by QINGCHUNBAO Pharmaceutical Co., Ltd. (B.N.: 0003132)( Hangzhou, Zhejiang Province).

**Experimental Groups:**

    (1) A Blank Control group, 3ml/kg physiological saline, n=5;
    (2) A Group of Herbesser Injection, 0.5ml/kg, n=5;
    (3) A Group of Radix Salviae Miltiorrhizae Injection, 0.6g/kg, n=5;
    (4) A Group of the composition of Radix Codonopsis and Radix Astragali 0.6g/kg, n=5;
    (5) A Group of the composition of Radix Codonopsis and Radix Astragali 1.2g/kg, n=5;
    (6) A Group of the composition of Radix Codonopsis and Radix Astragali 2.4g/kg, n=5.

The experimental medicaments were prepared to the same volume (50ml) with physiological saline, and injected through femoral vein at a speed of 5ml/min by the computer minim syringe pump (Type AJ-5803, Shanghai).

**Experimental Method:**

The animals were anaesthetized intravenously with pentobarbital sodium (30mg/kg, *i.v.*), and connected with an electric respirator after tracheal intubation. The chests were opened in the position of the 4th left rib to expose the heart. The pericardium was cut to make a pericardial bed. The laevorotatory branch of coronary artery and the root of aorta were separated and a probe of an electromagnetic flowmeter (Type MF-1100) was placed in to measure the blood flow of coronary artery and cardiac output. The tip of left ventricle was intubated and connected with a pressure energy transducer (MPU-0.5A). The internal pressure of left ventricle was measured with a carrier amplifier (AG-601 G), and the maximal ascending rate ($dp/dt_{max}$) of the internal pressure of left ventricle was measured with a differentiator (ED-601 G). The animals were intubated through the external jugular vein into the coronary vein sinus and also intubated through the carotid. The blood oxygen contents of coronary vein sinus and artery were measured using a blood oxygen instrument (Type AVL912, Swiss) to calculate the myocardial oxygen consumption. Then the femoral artery was intubated to measure the blood pressure of artery. And then with the Standard Limb Lead II of electrocardiogram, the heart rate and the related parameters of electrocardiogram were calculated. With the formula, other indexes of hemodynamics were calculated: cardiac stroke output, oxygen consumption index, cardiac index, resistance of coronary artery, total peripheral resistance and oxygen utility, etc. All the above-mentioned indexes were recorded by a multirunning physiological recorder (Type RM-6000, Japan Photoelectricity) synchronously.

After the operation and the observed indexes being stable, the values before medicament were recorded, and the experimental medicaments were then administered. The values were recorded at 5 minutes of the middle of the process of administration, immediately after, and 1, 3, 5, 10, 15, 30, and 60 minutes after administered medicaments. All the measured indexes and derived parameters were processed statistically. The self-comparisons of the actual measurement values within different time periods after and before administered medicaments were made. Using the t-Test, the changed rate percentages were compared to show the significance between the groups.

**Experimental Results:**

**The influence upon arterial blood pressure, heart rate and electrocardiogram of dogs**

The Herbesser Injection had an obvious effect of reducing blood pressure and slowing down heart rate after being administrated. In the 1.2g and 2.4g Crude Drug dose groups of the Composition of Radix Codonopsis and Radix Astragali, the arterial blood pressure was reduced both in the middle of administration and in a short period of time (1 minute) after administration. The Composition of Radix Codonopsis and Radix Astragali also slowed down the heart rate. The parameters of the PR wave, QRS wave, QT wave and T wave of the Electrocardiogram Standard II of animals were not obviously changed.

**The influence upon blood flow and resistance of coronary artery of dogs**

There were no obvious changes in the blood flow and resistance of coronary artery in the Group of physiological saline before and after *the administration of the* medicaments. In the 0.6g Crude Drug Group of the Composition of Radix Codonopsis and Radix Astragali, the blood flow of coronary artery was increased after medicament administration, and there was no obvious effect in the other two groups of the Composition. In all three dose groups of the Composition of Radix Codonopsis and Radix Astragali, the resistances of coronary artery were reduced. And in the process of medicaments administration, the resistance of coronary artery was *lowered* by about 15% and kept this low for continuously 60 minutes. Herbesser Injection, the calcium antagonist, also markedly increased the blood flow and the resistance of coronary artery.

**The influence upon contraction force and work of left ventricle**

There was no obvious difference in the contraction force and work of left ventricle of animals among all the experimental groups after medicaments administration.

**The influence upon the internal pressure and the maximal ascending speed of internal pressure in left ventricle of dog**

In the three dosage groups of the Composition of Radix Codonopsis and Radix Astragali, there was no obvious difference in the internal pressure and the maximal ascending speed ($dp/dt_{max}$) of internal pressure in left ventricle comparing to the Control group.

**The influence upon cardiac output, cardiac stroke output and total peripheral resistance of dogs**

In the 2.4g Crude drug/kg group of the Composition of Radix Codonopsis and Radix Astragali, the cardiac output was gradually increased and the most remarkable effect appeared after 5 minutes of administration. There was significant difference (P<0.05 - 0.01) comparing to the index before administration and that of the group of physiological saline. And at the same time, the resistance of outer periphery was obviously reduced. 6-Keto-PGFI markedly increased the cardiac stroke output and reduced the resistance of outer periphery.

**The influence upon blood oxygen contents of coronary artery and vein of dogs**

In the 2.4g Crude drug/kg group of the Composition of Radix Codonopsis and Radix Astragali, the blood oxygen content of animal coronary vein sinus was obviously increased during 5 - 15 minutes after medicament, and there was significant difference (P<0.05 - 0.001) from the one before administration and that of the group of physiological saline.

**The influence upon oxygen consumption, oxygen consumption index and oxygen utility of myocardium of dogs**

In the groups of the Composition of Radix Codonopsis and Radix Astragali, the oxygen consumption and oxygen utility of myocardium were not obviously changed. The oxygen consumption index of myocardium was obviously reduced in the process of and a short period of time after medicament administration.
In this experiment, the influence of the composition of Radix Codonopsis and Radix Astragali upon the cardiac hemodynamics and myocardial oxygen consumption of normally anaesthetized dogs was observed. Using the Herbesser Injection as the positive contrast medicament, the reliability and sensitivity of the experimental methods and the obtained indexes were proven.
It was shown in the experimental results that the Composition of Radix Codonopsis and Radix Astragali had the effects of expanding coronary artery and increasing the blood oxygen content of coronary vein sinus to improve the blood and oxygen supply of myocardium. At the same time, the Composition improved the work of left ventricle, increased cardiac output, and adjusted the cardiovascular compliance. It played certain adjusting and improving roles in the cardiovascular system and provided experimental evidences for the clinic treatment of ischemia heart disease.

**Reference Example 7:**

**The influence of the Composition of Radix Codonopsis and Radix Astragali upon thrombosis *in vitro* and blood viscosity of rats**

**Experimental Medicament:**

The composition of Radix Codonopsis and Radix Astragali: 5ml/piece, 2g Crude Drug/ml; Radix Salviae Miltiorrhizae Injection: 10ml/piece, 1.5g/ml, produced by QINGCHUNBAO Pharmaceutical Co., Ltd.( Hangzhou, Zhejiang Province) (B.N.: 0003132); Aspirin-DL-Lysine for Injection (0.9g/bottle, equaling to 0.5g aspirin): produced by LAIBILIN Pharmaceutical Co., Ltd. (Bengbu, Anhui Province) (B.N.: 000116); 0.9% Sodium Chloride Injection: produced by Beijing Double-crane Pharmaceutical Co., Ltd (B.N.: 000320332).

60 healthy male rats with weight of 248.9±16.9g were provided by the Medical Experimental Animal Center of Chinese Academy of traditional Chinese medicine with Certification No. 01-3067 of Medical Animals. The *in vitro* thrombosis Instrument, Type SDZ-A1, was produced by Electronic Instrument Factory of Wuxi, Jiangsu Province. The Blood Viscometer (Type LG-R-20) was produced by Beijing SHIDI Scientific Instrument Co.

## Experimental Methods:

The 60 rats were randomly divided into 6 groups (10 per group):

(1) A Control group , (0.9% Sodium Chloride Injection, 4ml/Kg);
(2) A High dose group of the composition of Radix Codonopsis and Radix Astragali (8g Crude Drug/kg);
(3) A Mid-Dose Group of the composition of Radix Codonopsis and Radix Astragali (4g Crude Drug/kg);
(4) A Low dose group of the composition of Radix Codonopsis and Radix Astragali (2g Crude Drug/kg);
(5) A Group of Radix Salviae Miltiorrhizae Injection (1.6g Crude Drug/kg);
(6) A Group of Aspirin-DL-Lysine for Injection (90mg/kg).

All the animal groups were intravenously injected the medicaments through caudal vein with the said dose of 4ml/kg, once per day for continuously 3 days. 30 minutes after the final injection, animals were anaesthetized with pentobarbital sodium (30.0mg/kg). 2ml blood was then drawn from the abdominal aorta for the measurement of the thrombosis *in vitro* and 3ml blood (heparinized) was drawn for the measurement of blood viscosity.

Measurement of the thrombuses *in vitro*: According to Chandler method *in vitro,* the blood was immediately filled and sealed in a rotating ring with the volume less than 1/2 ring (1.8ml). It was placed on a thrombosis Instrument and rotated for 10 minutes (with experimental temperature of 37°C). Then the thrombus was spilled out and washed with physiological saline. The length was measured and the wet quality was weighed. The thrombus bar was placed in an 80°C oven for 3 hours. It was weighed after the weight getting stable.

Measurement of the blood viscosity: 0.8ml blood was taken from the 3ml blood sample (heparinized) to measure the whole blood viscosity. The rest of the sample was centrifuged at 650 X g for 10 minutes. 0.8ml supernatant was taken to measure the blood plasma viscosity.

## Experimental Results:

**The influence upon thrombosis *in vitro* could be found in Table 12.**

**Table 12: The influence of the composition of Radix Codonopsis and Radix Astragali upon thrombosis of rats *in vitro* (X±D)**

| Group | Dos e (/kg) | Number of animals (n) | Thrombus | | |
|---|---|---|---|---|---|
| | | | Length (mm) | Wet weight (mg) | Dry weight (mg) |
| Control group | | 10 | 22.0±1.2 | 118.7±11.9 | 21.6±1.3 |
| Composition | 8g | 10 | 18.6±1.2*** | 98.0±11.8*** | 19.1±2.2** |
| Composition | 4g | 10 | 20.2±1.5** | 109.1±12.2 | 20.5±1.8 |
| Composition | 2g | 10 | 21.3±1.4 | 119.0±12.9 | 21.8±1.4 |
| Radix Salviae Miltiorrhizae injection | 1.6g | 10 | 20.4±1.2** | 106.1±7.8* | 20.1±1.2* |

(continued)

| Group | Dos e (/kg) | Number of animals (n) | Thrombus | | |
|---|---|---|---|---|---|
| | | | Length (mm) | Wet weight (mg) | Dry weight (mg) |
| Aspirin-DL-Ly sine | 90m g | 10 | 18.8±1.5*** | 100.1±12.5 ** | 18.9±1.4*** |

Noting: In comparison with the Control group: * P<0.05, ** P<0.01, *** P<0.001 As shown in Table 12, in comparison with the ones of the Control group, the length of thrombus was obviously shortened (P<0.001) in the High dose group of the composition of Radix Codonopsis and Radix Astragali, and the wet and dry weights of thrombus was obviously lightened (P<0.01); the length of thrombus was obviously shortened (P<0.01) in the Mid-Dose group of the composition of Radix Codonopsis and Radix Astragali, and the wet and dry weights of thrombus had a lightening trend; the length and the wet and dry weights of thrombus of the low dose group showed no significant difference. In comparison with the Control group, the length of thrombus was obviously shortened (P<0.01) in the group of Radix Salviae Miltiorrhizae injection, and the wet and dry weights of thrombus was obviously lightened (P<0.05); the length of thrombus was obviously shortened (P<0.001) in the group of Aspirin-DL-Lysine, and the wet and dry weights of thrombus was obviously lightened (P<0.01 - 0.001).

**The influence upon the blood viscosity could be found in Table 13.**

**Table 13: The influence of the composition of Radix Codonopsis and Radix Astragali upon the blood viscosity of rats (n=10, X±D)**

| Group | Dose (mg/k g) | Number of animals (n) | Whole blood viscosity (CP) | | | | |
|---|---|---|---|---|---|---|---|
| | | | High shearing $(200S^{-1})$ | Middle shearing $(100S^{-1})$ | Middle shearing $(30S^{-1})$ | Low shearing $(5S^{-1})$ | Blood viscosity $(100S^{-1})$ |
| Control Group | | 10 | 4.23±0. 86 | 5.41±1. 64 | 6.68±2. 26 | 990±4.2 1 | 2.69±1. 33 |
| Composition | 8g | 10 | 3.65±0. 39 | 4.08±0. 42* | 5.11±0. 68* | 6.89±1.1 3* | 2.41±1. 18 |
| Composition | 4g | 10 | 3.69±0. 64 | 4.19±0. 95 | 5.54±1. 94 | 8.13±4.1 4 | 2.60±1. 47 |
| Composition | 2g | 10 | 4.16±0. 80 | 4.67±0. 94 | 6.00±1. 33 | 8.48±2.1 1 | 1.98±1. 21 |
| Radix Salviae Miltiorrhizae injection | 1.6g | 10 | 3.35±0. 32** | 3.72±0. 38** | 4.68±0. 55* | 6.43±0.8 8* | 2.12±1. 20 |
| Aspirin-DL- Lysine | 90mg | 10 | 3.37±0. | 3.81±0. | 4.71±0. | 6.35±1.0 | 2.38±1. |

(continued)

| Group | Dose (mg/k g) | Number of animals (n) | Whole blood viscosity (CP) | | | | |
|---|---|---|---|---|---|---|---|
| | | | High shearing (200S⁻¹) | Middle shearing (100S⁻¹) | Middle shearing (30S⁻¹) | Low shearing (5S⁻¹) | Blood viscosity (100S⁻¹) |
| | | | $58^*$ | $91$ | $95^*$ | $3^*$ | $12$ |

**Noting:** In comparison with the Control group: $^*$ $P<0.05$, $^{**}$ $P<0.01$. As shown in Table 13, in the High dose group of the composition of Radix Codonopsis and Radix Astragali, the whole blood viscosities of rats under shear-rates of $10OS^{-1}$, $30S^{-1}$, and $5S^{-1}$ was obviously lower than the one of the Control group ($P<0.05$), and it also had a descending trend under the shear-rate of $200S^{-1}$. In the Mid-Dose group of the composition of Radix Codonopsis and Radix Astragali, the whole blood viscosities of rats had a descending trend under shear-rates of $100S^{-1}$, and there was no significant difference under high and low shearing in comparison to that of the Control group. In the Low dose group of the Composition of Radix Codonopsis and Radix Astragali, the whole blood viscosities under all the shear-rates had no significant difference comparing to that of the Control group. In the Group of Radix Salviae Miltiorrhizae Injection, the whole blood viscosities under all the shear-rates were obviously lower than that of the Control group ($P<0.05$ - $0.01$). In the Group of Aspirin-DL-Lysine for Injection, the whole blood viscosities of rats under all the shear-rates were lower than that of the Control group. There was no significant difference in the blood serum viscosity of all the medicaments administration groups comparing to the Control group.

It was shown in the above-mentioned results that with 3 days of continuous intravenously injection of medicaments to rats, the Composition of Radix Codonopsis and Radix Astragali with a dose of 8g Crude Drug/kg markedly shortened the length of thrombus ($P<0.001$), lightened the wet and dry weight of thrombus ($P<0.01$), and reduced the whole blood viscosity under the shear-rates of $100S^{-1}$, $30S^{-1}$, and $5S^{-1}$ ($P<0.05$). It suggested that the Composition of Radix Codonopsis and Radix Astragali had effects of inhibiting thrombosis and reducing blood viscosity.

**Reference Example 8:**

**The influence of the Composition of Radix Codonopsis and Radix Astragali upon platelet aggregation of rabbits**

**Experimental Medicament:**

The composition of Radix Codonopsis and Radix Astragali was 5ml/piece and 2g Crude Drug/ml. Radix Salviae Miltiorrhizae injection was 10ml/piece, 1.5g/ml and produced by QINGCHUNBAO Pharmaceutical Co., Ltd.( Hangzhou, Zhejiang Province) (B.N.: 0003132). 0.9% Sodium Chloride Injection was produced by Beijing Double-crane Pharmaceutical Co., Ltd (B.N.: 000320332). Aspirin-DL-Lysine for Injection (0.9g/bottle, equaling to 0.5g aspirin) was produced by LAIBILIN Pharmaceutical Co., Ltd.(Bengbu, Anhui Province) (B.N.: 000116). Adenosine diphosphate (ADP) disodium salt was produced by Shanghai Biochemistry Research Institute (B.N.: 9209258) and prepared with physiological saline into a 1.0mM/L solution storing at 4°C. Arachidonic acid (AA) was produced by Fluka AG, and prepared temporarily with 1.0mM/L NaOH into sodium salt with concentration of 5.0g/L. Collagen (100 μg/ml) was produced by KOKEN Co.

**Experimental Animals:**

48 healthy male Japanese flap-eared white rabbits with weight of $2.75\pm0.15$kg were provided by the Experimental Animals Center of China Veterinary Medicaments Supervision Institute with Qualification Number of 004 (1999). The platelet aggregation instrument (Type BS634) was produced by Beijing Biochemical Instrument Factory.

**Experimental Method:**

Before administration, the artery in ears was punctured and the blood was taken to measure the platelet aggregation rate. According to the level of platelet aggregation and the body weight, the 48 rabbits were randomly divided into 6 groups (8 rabbits per group):

(1) A Control group, (0.9% sodium chloride Injection, 2.5ml/kg);
(2) A High dose group of the composition of Radix Codonopsis and Radix Astragali (5g Crude Drug/kg);
(3) A Mid dose group of the composition of Radix Codonopsis and Radix Astragali (2.5g Crude Drug/kg);
(4) A Low dose group of the composition of Radix Codonopsis and Radix Astragali (1.25g Crude Drug/kg);

(5) A Group of Radix Salviae Miltiorrhizae Injection (1g Crude Drug/kg);
(6) A Group of Aspirin-DL-Lysine for Injection (45mg/kg).

The administration groups were administered medicaments intravenously through ear edges with the said dosages, and the Control group was injected with equivalent volume of 0.9% sodium chloride, once per day for continuously 3 days. After the final medicaments administration, the artery in ears was punctured, the blood sample was taken and the platelet aggregation rate was measured.

Measurement Method of Platelet Aggregation Rate:

The artery in ears was punctured with a siliconized injector to take blood sample, which was anticoagulated with the citron sodium solution (blood:anticoagulant = 9:1). The blood sample solution was centrifuged at 200 X g for 8 minutes, and the supernatant, that is, the Platelet Rich Plasma (PRP) was taken. The rest of the solution was centrifuged at 2200 x g for 10 minutes, and the top clear part, that is, the Platelet Poor Plasma (PPP) was taken. The count of Platelets in PRP was about $4.0 \times 10^5/mm^3$. According to the Born Turbidimetry, the turbidity tube with 200 $\mu$l PRP and a small magnetic stick was placed in the platelet aggregation instrument and kept at 37°C for one minute. After PPP being standardized, an inducer was added with stirring to induce the aggregation. The end concentration of the inducer is: 47.6 $\mu$M/L (ADP), 782.0 $\mu$M/L (AA), and 4.8 mg/ml (Collagen). The influence of the medicaments upon the Platelet Aggregation was analyzed according to the aggregation curves and maximal aggregation rate printed by the instrument. The calculating formula for maximal aggregation rate was as follows:

$$\text{Maximal aggregation rate} = \frac{\text{Transmittance after PRP Aggregation - Transmittance before PRP Aggregation}}{\text{Transmittance of PPP - Transmittance before PRP Aggregation}} \times 100\%$$

The experimental results could be found in Table 14.

**Table 14: The influence of the composition of Radix Codonopsis and Radix Astragali upon the Platelet Aggregation Rate of Rabbits**

| Inducer | Group | Dosage /kg | Animal No. | Aggregation Rate (%, X±SD) | | |
| --- | --- | --- | --- | --- | --- | --- |
| | | | | Before administration | After administration | Value Difference |
| ADP | Control Group | | 8 | 65.23±8.76 | 66.42±8.64 | 1.19±7.09 |
| | Composition | 5g | 8 | 66.56±6.56 | 59.08±6.26 | -7.48±8.18* |
| | Composition | 2.5g | 8 | 65.23±8.36 | 60.90±6.93 | -4.33±11.95 |
| | Composition | 1.25g | 8 | 65.26±7.98 | 61.13±3.82 | -4.13±8.67 |
| | Radix Salviae Miltiorrhizae Injection | 1g | 8 | 65.72±10.96 | 57.57±6.43 | -8.15±9.48* |
| | Aspirin-DL-Lysine | 45mg | 8 | 66.62±7.09 | 57.78±6.01 | 8.85±6.37 |
| AA | Control Group | | 8 | 65.67±8.63 | 67.28±10.06 | 1.61±9.61 |
| | Composition | 5g | 8 | 68.00±5.42 | 55.46±6.94 | -12.54±6.01** |
| | Composition | 2.5g | 8 | 67.19±9.24 | 57.95±4.75 | -9.25±9.57* |
| | Composition | 1.25g | 8 | 64.62±7.22 | 63.13±9.46 | -1.50±10.58 |

(continued)

| Inducer | Group | Dosage /kg | Animal No. | Aggregation Rate (%, X±SD) | | |
|---|---|---|---|---|---|---|
| | | | | Before administration | After administration | Value Difference |
| | Radix Salviae Miltiorrhizae Injection | 1g | 8 | 63.99±5.46 | 54.86±4.60 | -9.12±8.29* |
| | Aspirin-DL-Lysine | 45mg | 8 | 63.90±7.06 | 0.24±0.69 | -63.65±7.05*** |
| Collagen | Control Group | | 8 | 68.82±6.79 | 68.00±10.27 | -0.82±9.95 |
| | Composition | 5g | 8 | 68.11±5.88 | 64.84±9.19 | -3.26±10.61 |
| | Composition | 2.5g | 8 | 69.36±7.04 | 66.74±6.58 | -2.62±6.41 |
| | Composition | 1.25g | 8 | 68.01±6.40 | 66.05±7.89 | -1.96±5.77 |
| | Radix Salviae Miltiorrhizae Injection | 1g | 8 | 67.06±7.67 | 63.44±3.95 | -3.61±9.69 |
| | Aspirin-DL-Lysine | 45mg | 8 | 66.49±3.83 | 45.29±5.72 | -21.19±4.24*** |

**Noting:**

1. In comparison with the Control group: * P<0.05, **** P<0.01, and *** P<0.001.

2. Value Difference = Aggregation rate after administration - Aggregation rate before administration.

Results obtained from Table 14:

(1) When ADP was used to induce aggregation, there was no obvious difference in the platelet aggregation rates among all the groups before medicaments administration. After administration, the platelet aggregation rates were obviously lower in the High dose group of the composition of Radix Codonopsis and Radix Astragali and the Group of Radix Salviae Miltiorrhizae Injection than in the Control group (P<0.05). The platelet aggregation rates of the Group of Aspirin-DL-Lysine were obviously lower than that of the Control group (P<0.01). In the Mid dose and Low dose groups of the composition of Radix Codonopsis and Radix Astragali, there was a descending trend in platelet aggregation rate comparing to that of the Control group.

(2) When AA was used to induce aggregation, there was no obvious difference in the platelet aggregation rates among all the groups before medicaments administration. After administration, the platelet aggregation rates were obviously lower in the High dose and Mid dose groups of the composition of Radix Codonopsis and Radix Astragali than in the Control group (P<0.05 - 0.01). In the Low dose group of the composition of Radix Codonopsis and Radix Astragali, there was no distinct difference in the platelet aggregation rate comparing to the Control group. The platelet aggregation rate of the Group of Radix Salviae Miltiorrhizae Injection was obviously lower than that of the Control group (P<0.05). And the platelet aggregation rate of the Group of Aspirin-DL-Lysine was obviously lower than that of the Control group (P<0.001).

(3) When Collagen was used to induce aggregation, there was no obvious difference in the platelet aggregation rates among all the groups before medicaments administration. After administration, the platelet aggregation rate of the Group of Aspirin-DL-Lysine was obviously lower than that of the Control group (P<0.001). And for the Platelet Aggregation Rates of all the other groups, there was no significant difference in comparison with that of the Control group. The above-mentioned results showed that after being continuously injected via *i.v.* for 3 days, the Composition of Radix Codonopsis and Radix Astragali (5g Crude Drug/kg) obviously reduced the platelet aggregation rate of rabbits (P<0.05 ~ 0.01), which was induced by adenosine(ADP) and arachidonic acid(AA) appeared. The Composition of Radix Codonopsis and Radix Astragali (2.5g Crude Drug/kg) obviously reduced the platelet aggregation rate of rabbits (P<0.05) induced by AA. It suggested that the Composition of Radix Codonopsis and Radix Astragali had effect of inhibiting the platelet aggregation.

**Experimental Example 8:**

**1. Experimental medicaments:**

The composition of Radix Codonopsis and Radix Astragali was the extracted Composition of Radix Codonopsis and Radix Astragali prepared in accordance with the above-mentioned implementation examples, in which each 20ml composition contained 3.25g equivalent amount of Radix Codonopsis and Radix Astragali. The composition was water bathed, concentrated to 2ml - 1ml and sterilized by circulating steam for 40 minutes.
LPS: powder, USA Sigma Co., diluted with sterile physiological saline before usage.
Dexamethasone (Chengdu Pharmaceutical Factory No. 1)

**2. Experimental Animal:**

The Wistar rats were provided by Huaxi College Animal Center of Sichuan University with Microorganism Control Level II.

**3. Experimental Methods:**

The 60 rats were randomly divided into 4 groups, that is, normal Control group, modeling group (LPS+NS Group), Dexamethasone intervening (treatment) group (LPS+DEX Group) and Composition of Radix Codonopsis and Radix Astragali Injection intervening (treatment) group (LPS+SQ Group). And each of the modeling group, the LPS+DEX Group and LPS+SQ Group was again divided into 3 time phasing groups, that is, a one-hour group, a two-hour group, and a four-hour group. There are 10 groups in total, 6 mice per group in this experiment.

**Experimental Methods:**

**1. Preparation Work**

3 days before the experiment, all the appliances, vessels and transplant agents were sterilized with high temperature and high pressure, and dried for future usage. The experimental appliances and reagents, which were used to extract the total RNA, were dipped in DEPC solution overnight, sterilized and dried.

**2. Preparation of ALI Model and the method of administration**

1 week before modeling, the 18 rats of the LPS+SQ Group were injected daily with 2ml extracted Composition of Radix Codonopsis and Radix Astragali abdominally as pretreatment. The 18 rats of the LPS+DEX Group were injected abdominally with 50mg/kg Dexamethasone 2 hours before modeling. The 18 rats of the LPS+NS Group were injected with physiological saline 2 hours before modeling. Except for the normal Control group, all the three above-mentioned groups were injected with LPS (4mg/kg) in the caudal vein.

**3. Sampling**

After all the animals being anaesthetized with chloral hydrate through abdominal injection at the determined time phasing point, 2ml blood was sampled from the carotid, and centrifuged at 800gr/min for 8 minutes. The supernatant was extracted and placed at -20°C in a refrigerator for future usage. The chest was opened and the lower lobes of the right lung were sampled. They were placed in the EP tubes treated with DEPC solution and immediately put at -70°C in a refrigerator for the total RNA extraction. The upper lobes of the right lung were sampled and dipped in 10% formaldehyde solution to fix. The left lungs were taken for the ratio of wet/dry weight measurement. 4. At the corresponding timing point, the animal lung tissues were taken and observed with human eyes and under the microscope using HE Staining Method. The ratio of tissues wet/dry weight was also measured.

**Experimental Results:**

The symptoms of tachypnea, lips cyanosis, mental wilting, little movement and little eating occurred in all the experimental animals injected with LPS in the caudal veins, especially those animals of LPS+NS Group at every time phasing points. However, the symptoms were relatively mild and lasted relatively short period of time in LPS+DEX and LPS+SQ Groups. All the animals survived at all the time phasing points set by this experiment.

### General Observation

In the normal Control group, the lung tissues appeared white and reddish, soft and elastic. In the one-hour LPS+NS Group, congestion and edema were observed in the lungs, and the spotted petechiae were occasionally observed on the surface. In the two-hour group, the surface of lungs presented in dark red with obvious congestion, edema, flake-shaped petechiae and bleeding focus. The conditions of the four-hour group were generally the same as that of the two-hour group. The symptoms observed in Group LPS+DEX and Group LPS+SQ appeared mitigated to different extent at the same time phasing points, in comparison to that observed in Group LPS+NS.

### Observation with an optical microanalyser

The results were basically consistent to the general observation. The lung tissues of the normal Control group were clear in structure with thin pulmonary alveoli wall. There were also infiltrated inflammatory cells and edemata in the widened interstitial lung. It was classified as Light in the Pathological Classification of acute lung injury. The lung cell injury of the two-hour group was the most obvious. The remarkable widened interstitial lung was visible with effusion and edema. The transparent membranes formed, pulmonary alveoli bled and large amount of inflammatory cells were infiltrated. It was classified as Middle - Severe in the ALI Pathological Classification. The injury of lung cells of the four-hour group was basically the same as that of the two-hour group, and classified mostly as Middle in the ALI Pathological Classification. In Group LPS+NS, the injury was mitigated to some extent at the corresponding time phasing points. The Pathological Classification of all the groups could be found in Table 15.

**Table 15: Pathological Classification of lung injury for all groups**

| Group | No. of cases | 1-hour | | | 2-hour | | | 4-hour | | |
|---|---|---|---|---|---|---|---|---|---|---|
| | | Light | Middle | Severe | Light | Middle | Severe | Light | Middle | Severe |
| Normal Group | 6 | -- | -- | -- | -- | -- | -- | -- | -- | -- |
| LPS+NS | 18 | 5 | 1 | 0 | 0 | 3 | 3 | 0 | 4 | 2 |
| LPS+DEX | 18 | 6 | 0 | 0 | 4 | 2 | 0 | 6 | 0 | 0 |
| LPS+SQ | 18 | 6 | 0 | 0 | 3 | 6 | 0 | 5 | 1 | 0 |
| The results of the sighed-rank sum test were as follows: There was no significant difference in pathological changes at 1-hour (P>0.05) phase point between the Modeling group and other intervention groups. However, there was significant difference in pathological changes at 2-hour and 4-hour (p<0.05). Comparing among all intervention groups at different timing points respectively, there was significant difference in Group LPS+NS (p<0.05); and the difference in Groups LPS+DEX and LPS+SQ had no statistical meaning (p>0.05). | | | | | | | | | | |

### Changes of lung wet/dry (W/D) weight ratio

The lung W/D ratios of Group LPS+NS at all time phasing points were obviously higher than that of the normal Control group (p<0.05). The ones of Groups LPS+DEX and LPS+SQ were reduced at the same time phasing point (P<0.05). Data were referred to Table 16.

**Table 16: Change of Lung W/D Ratio for all the groups**

| Groups | No. of cases | 1 h | 2h | 4h |
|---|---|---|---|---|
| Normal | 6 | $4.11 \pm 0.29$ | -- | -- |
| LPS+NS | 18 | $5.46 \pm 0.37^{*}$ | $5.53 \pm 0.03^{*}$ | $5.68 \pm 0.14^{*}$ |
| LPS+DEX | 18 | $4.82 \pm 0.23^{*}\Delta$ | $4.59 \pm 0.19^{*}\Delta\#$ | $4.57 \pm 0.10^{*}\Delta\#$ |

(continued)

| Groups | No. of cases | 1 h | 2h | 4h |
|---|---|---|---|---|
| LPS+SQ | 18 | $5.06 \pm 0.24 * \Delta$ | $4.94 \pm 0.56 * \Delta$ | $4.62 \pm 0.41 * \Delta$ |

**Noting:** *: In comparison with the normal Control group, $p<0.05$; $\Delta$: In comparison with Group LPS+NS at the same time phasing point, $p<0.05$; #: Comparison between all the time phasing points and 1-h time phasing points in the intervention groups, $p<0.05$.

It has been proven in further animal experiments that the Composition of Radix Codonopsis and Radix Astragali can obviously reduce the pathological changes during the acute lung injury, that is, the pathological changes of acute lung injury caused by the lipopolysaccharide. It can also obviously mitigate the edema of lung tissues. In comparison with the untreated group, the ratio of W/D weight of lung tissues is obviously reduced. When the Composition of Radix Codonopsis and Radix Astragali is used alone to treat the acute lung injury, the treating effect is almost the same as that of when using dexamethasone alone.

In this invention, the traditional medicine Radix Codonopsis and Radix Astragali are comprehensively and systematically researched. Their effective parts are clearly defined and function mechanism described. The special extraction technology has been designed. The impurities are removed as much as possible while the effectiveness of the medicament is kept, so that the dosage and the toxic side-effect can be reduced. The method is simple with strong practicability, creativeness and elicitation. It can be a strong base for the modernization of the Traditional Chinese medicine.

## Claims

1. Use of a composition of Radix Codonopsis and Radix Astragali for the preparation of a medicament for preventing and treating the acute pulmonary injury, wherein the range of the weight ratio of the Radix Codonopsis and Radix Astragali is from 0.5:1 to 1:0.5.

2. The use according to Claim 1, **characterized in that** every gram of the composition comprises no less than 0.325 g of solid.

3. The use according to Claim 1 or 2, **characterized in that** the weight ratio of Radix Codonopsis and Radix Astragali is 1:1.

4. The use according to of Claim 1 or 2, **characterized in that** the medicament further comprises the Chinese Medicines of invigorating QI and enriching blood.

5. The use according to Claim 4, **characterized in that** the Chinese Medicines of invigorating QI and enriching blood are Radix Angelicae Sinensis, Radix rehmanniae Praeparata, Radix Polygoni Multiflori, Rhizoma Atractylodis Macrocephalae, Rhizoma Dioscoreae or their combinations.

6. The use according to any one of Claims 1 to 5, **characterized in that** the composition can be used in the form of injection, tablet, pill, capsule, granule, solution, suspension or emulsion.

7. The use according to any one of Claims 1 to 5, **characterized in that** the effective dosage range of the composition is 58 - 70mg/kg (body weight)/day.

8. A method of preparing a composition of Radix Codonopsis and Radix Astragali, comprising the following steps of:

   a) removing impurities from Radix Codonopsis and Radix Astragali, then making them into herbal pieces prepared for decoction;
   b) taking Radix Codonopsis and Radix Astragali in a certain weight ratio and washing them with deionized water;
   c) adding a certain amount of deionized water stepwise according to the mass of Radix Codonopsis and Radix Astragali, heating and extracting one to three times to obtain the pharmaceutical extract;
   d) condensing the pharmaceutical extract and obtaining the condensed liquid;
   e) adding proposal amount of ethanol, depositing normally, filtrating, recovering the ethanol from the filtered liquid, and condensing to dry to obtain the extracted composition of Radix Codonopsis and Radix Astragali.

9. The method according to Claim 8, **characterized in that** the amount of added water is eight times volume for the first time, decocting for one hour; and the amount of added water is six times volume for the second time, decocting for 0.5 hour in Step c) of extracting the medicaments by decocting with water.

10. The method according to Claim 8, **characterized in that** the content of ethanol is 65%-80% of the total weight for the first time; and the content of ethanol is no less than 80% of the total weight for the second time in Step e) of depositing the medicaments by adding ethanol.

## Patentansprüche

1. Verwendung einer Zusammensetzung aus Radix Codonopsis und Radix Astragali für die Herstellung eines Medikaments zum Vorbeugen und Behandeln des akuten Lungenschadens, worin der Bereich des Gewichtsverhältnisses von Radix Codonopsis und Radix Astragali von 0,5:1 bis 1:0,5 ist.

2. Die Verwendung gemäß Anspruch 1, **dadurch gekennzeichnet, dass** jedes Gramm der Zusammensetzung nicht weniger als 0,325 g Feststoff umfasst.

3. Die Verwendung gemäß Anspruch 1 oder 2, **dadurch gekennzeichnet, dass** das Gewichtsverhältnis von Radix Codonopsis und Radix Astragali 1:1 ist.

4. Die Verwendung gemäß Anspruch 1 oder 2, **dadurch gekennzeichnet, dass** das Medikament weiterhin die chinesischen Arzneimittel zum Kräftigen des QI und Bereichern von Blut umfasst.

5. Die Verwendung gemäß Anspruch 4, **dadurch gekennzeichnet, dass** die chinesischen Arzneimittel zum Kräftigen des QI und Bereichern von Blut Radix Angelicae Sinensis, Radix rehmanniae Praeparata, Radix Polygoni Multiflori, Rhizoma Atractylodis Macrocephalae, Rhizoma Dioscoreae oder deren Kombinationen sind.

6. Die Verwendung gemäß einem der Ansprüche 1 bis 5, **dadurch gekennzeichnet, dass** die Zusammensetzung in Form einer Injektion, Tablette, Pille, Kapsel, Körnchen, Lösung, Suspension oder Emulsion verwendet werden kann.

7. Die Verwendung gemäß einem der Ansprüche 1 bis 5, **dadurch gekennzeichnet, dass** der wirksame Dosierungsbereich der Zusammensetzung 58 bis 70 mg/kg (Körpergewicht)/Tag beträgt.

8. Ein Verfahren zum Herstellen einer Zusammensetzung aus Radix Codonopsis und Radix Astragali umfassend die folgenden Schritte:

   a) Entfernen von Verunreinigungen aus Radix Codonopsis und Radix Astragali, anschließend Überführen in pflanzliche Stücke, die zum Absieden vorbereitet werden;
   b) Aufnehmen des Radix Codonopsis und Radix Astragali in einem bestimmten Gewichtsverhältnis und Waschen mit entionisiertem Wasser;
   c) schrittweises Zufügen einer bestimmten Menge an entionisiertem Wasser gemäß der Masse von Radix Codonopsis und Radix Astragali, Erhitzen und ein- bis dreimaliges Extrahieren, um den pharmazeutischen Extrakt zu erhalten;
   d) Kondensieren des pharmazeutischen Extraktes und Erhalten der kondensierten Flüssigkeit;
   e) Zufügen einer vorgeschlagenen Menge an Ethanol, normal Absetzen lassen, Filtrieren, Zurückgewinnen des Ethanols aus der filtrierten Flüssigkeit und Kondensieren bis zur Trockne, um die extrahierte Zusammensetzung aus Radix Codonopsis und Radix Astragali zu erhalten.

9. Das Verfahren gemäß Anspruch 8, **dadurch gekennzeichnet, dass** die Menge des zugefügten Wassers das achtfache Volumen beim ersten Mal beträgt, Absieden für eine Stunde; und die Menge an zugefügtem Wasser beträgt das sechsfache Volumen beim zweiten Mal, Abkochen für 0,5 Stunden in Schritt c) des Extrahierens des Medikaments durch Absieden mit Wasser.

10. Das Verfahren gemäß Anspruch 8, **dadurch gekennzeichnet, dass** der Gehalt an Ethanol 65-80 % des Gesamtgewichts beim ersten Mal beträgt; und der Gehalt an Ethanol ist nicht weniger als 80 % des Gesamtgewichtes beim zweiten Mal in Schritt e) beim Absetzen lassen des Medikamentes durch Zugabe von Ethanol.

**Revendications**

1. Utilisation d'une composition de Radix Codonopsis et Radix Astragali pour la préparation d'un médicament destiné à la prévention et au traitement d'une lésion pulmonaire aiguë, dans laquelle la plage du rapport en poids de Radix Codonopsis et Radix Astragali est de 0,5:1 à 1:0,5.

2. Utilisation selon la revendication 1,
   **caractérisée en ce que** chaque gramme de la composition ne comprend pas moins de 0,325 g de solide.

3. Utilisation selon la revendication 1 ou 2,
   **caractérisée en ce que** le rapport en poids de Radix Codonopsis et Radix Astragali est de 1:1.

4. Utilisation selon la revendication 1 ou 2,
   **caractérisée en ce que** le médicament comprend en outre des médicaments chinois consistant à revigorer le QI et à enrichir le sang.

5. Utilisation selon la revendication 4,
   **caractérisée en ce que** les médicaments chinois consistant à revigorer le QI et à enrichir le sang sont constitués de Radix Angelicae Sinensis, Radix rehmanniae Praeparata, Radix Polygoni Multiflori, Rhizoma Atractylodis Macrocephalae, Rhizoma Dioscoreae ou leurs combinaisons.

6. Utilisation selon l'une quelconque des revendications 1 à 5, **caractérisée en ce que** la composition peut être utilisée sous la forme d'injection, de comprimé, de pilule, de capsule, de granule, de solution, de suspension ou d'émulsion.

7. Utilisation selon l'une quelconque des revendications 1 à 5, **caractérisée en ce que** la plage posologique efficace de la composition est 58 à 70 mg/kg (poids corporel)/jour.

8. Procédé de préparation d'une composition de Radix Codonopsis et Radix Astragali, comprenant les étapes suivantes consistant à :

   a) retirer les impuretés de Radix Codonopsis et Radix Astragali, puis les mettre sous forme de fragments d'herbe préparés pour une décoction ;
   b) prendre Radix Codonopsis et Radix Astragali en un certain rapport en poids et les laver avec de l'eau désionisée ;
   c) ajouter une certain quantité d'eau désionisée par étape selon la masse de Radix Codonopsis et Radix Astragali, chauffer et extraire une à trois fois pour obtenir l'extrait pharmaceutique ;
   d) concentrer l'extrait pharmaceutique et obtenir le liquide concentré ;
   e) ajouter une quantité envisagée d'éthanol, laisser déposer normalement, filtrer, récupérer l'éthanol du liquide filtré et concentrer jusqu'à siccité pour obtenir la composition extraite de Radix Codonopsis et Radix Astragali.

9. Procédé selon la revendication 8, **caractérisée en ce que** la quantité d'eau ajoutée est huit fois le volume pour la première fois, en faisant une décoction pendant une heure ; et la quantité d'eau ajoutée est six fois le volume pour la seconde fois, en faisant une décoction pendant 0,5 heure dans l'Etape c) d'extraction des médicaments par décoction avec de l'eau.

10. Procédé selon la revendication 8, **caractérisé en ce que** la teneur en éthanol est de 65 % à 80 % du poids total pour la première fois ; et la teneur en éthanol n'est pas inférieure à 80 % du poids total pour la seconde fois dans l'Etape e) de dépôt des médicaments par ajout d'éthanol.

Radix Codonopsis

Radix Astragali

Water
extracting

condensing

Extract

Condensed
liquid

Ethanol deposited Twice
Filtered and recovered ethanol

Extract of Radix Codonopsis
and Radix Astragali

**Fig. 1**